# EUROPEAN PATENT SPECIFICATION

(11) **EP 3 669 906 B1**
(45) Date of publication and mention of the grant of the patent: **20.09.2023**
(21) Application number: 18215163.9
(22) Date of filing: 21.12.2018
(51) Int. Cl.: A61M 5/142, A61M 5/14

(54) **SKIN-MOUNTABLE MEDICAL DEVICE**
AUF DER HAUT ANBRINGBARE MEDIZINISCHE VORRICHTUNG
DISPOSITIF MÉDICAL POUVANT ÊTRE MONTÉ SUR LA PEAU

(43) Date of publication of application: 24.06.2020
(73) Proprietor: Roche Diabetes Care GmbH, 68305 Mannheim (DE); F. Hoffmann-La Roche AG, 4070 Basel (CH)
(72) Inventor: ARNOLD, Marc, 68305 Mannheim (DE); FREITAG Christian, 68305 Mannheim (DE); RASMUSSEN Mads Bjoern, 68305 Mannheim (DE)
(74) Representative: Pfiz, Thomas

(56) References cited:
- US-A1- 2017 224 912
- US-B1- 6 302 866
- US-B2- 9 248 232

## Description

The invention relates to a skin-mountable medical device comprising a mounting plate having a bottom side attachable to the skin of a user preferably via an adhesive pad, a medical module, in particular an analyte sensing device or a drug administration pump, and a snap connector adapted to releasably attach the medical module on the upper side of the mounting plate opposite to the bottom side.

Such medical device is known from WO 2009/125398 A2. There, the device comprises a skin-adherable cradle unit and a fluid dispensing unit, wherein the cradle unit has two latches and the dispensing unit is snap-fitted by operation of the latches 32, 34. For this purpose, the latches are configured as hook-like protrusions which are accommodated in complementary shaped grooves of the dispensing unit. However, WO 2009/125398 A2 fails to disclose details for user handling of the latches, and the latches appear susceptible to breakage upon insertion of the dispensing unit. Such breaking connections may lead to a loss of the medical device, which may even go unnoticed by the patient. US9248232 and US2017224912 also describe prior art medical devices.

On this basis, the object of the invention is to further improve the known medical devices and to provide an easy-to-use design which simplifies user manipulation while avoiding assembly failures.

The combination of features stated in the independent claim is proposed to achieve this object. Advantageous embodiments and further developments of the invention are derived from the dependent claims.

The invention is based on the idea of improving user interaction by avoiding interfering contours or contact in the mechanical movement paths. Accordingly, it is proposed that wherein the snap connector has a release flap that can be actuated or pushed by the user to detach the medical module, wherein the release flap projects outwardly from the mounting plate and has an outer portion which at its bottom side maintains a clearance to avoid an interfering skin contact when used or pressed. In this way, the release flap is allowed to move prior to a skin contact, and the user is prevented from applying excessive forces against a skin abutment which may lead to material failure. Moreover, the effort and force for insertion of the medical module is reduced, as no movable part of the snap connector comes into skin contact, thus also avoiding a bending or deformation of the mounting plate. As a result, the user-device-interaction is improved, and the intended medical success is further supported.

Advantageously, where the bottom side of the mounting plate lies in a plane or is generally planar, the outer portion of the release flap should be arranged at a clearance distance of at least 2 mm, preferably at least 5 mm above this plane. In this way, there is provided sufficient tolerance for unimpeded movement of the flap.

In another advantageous configuration, where the release flap has an inner portion connected to the mounting plate, the distance of the inner portion to a plane which is defined by the bottom side of the mounting plate (or, in use, by the skin) should be smaller than the distance of the outer portion to said plane.

In this connection it is further advantageous when the bottom sides of the mounting plate and of the release flap each define a plane, and wherein said planes include an acute angle.

Preferably, said acute angle is at least 5°, preferably at least 10°.

In another advantageous embodiment, the bottom side of the release flap runs parallel to the bottom side of the mounting plate. In such a parallel configuration, the release flap may be directly attached to an upstanding part of a latch of the snap connector.

A particular advantageous embodiment further comprises that the release flap has an upstanding circumferential rim which in use faces away from the skin. Thereby, the user receives a haptic feedback when pressing his finger against the flap. Furthermore, the rim facing upwards avoids unintended slipping of the finger and thereby supports higher pressing forces.

According to the invention, the snap connector has a latch that is connected to the mounting plate and engages with the medical module in a locking position, wherein the latch can be tilted outward by actuation of the release flap so as to disengage from the medical module in a release position.

For providing a mechanically secure connection, the latch has a hook portion which is adapted to snap into a recess of the medical module.

In order to support the application of the medical module onto the mounting plate, the snap connector has an inlet chamfer which is construed to guide the medical module into a locking position, wherein the inlet chamfer is arranged at an acute angle with respect to the bottom side of the mounting plate. Another possibility for facilitating handling and improving joining provides that a securing bracket is provided for attaching the medical module, wherein the securing bracket and the snap connector are arranged at opposite ends of the mounting plate.

In still another advantageous configuration, the mounting plate and the release flap are formed as an integral, preferably injection-moulded plastic part.

The medical device according to any of claims 1 to 12, In an advantageous configuration for drug administration, a fitting can be arranged on the bottom plate to provide a skin access preferably for a cannula assembly of the medical module.

In the following, the invention is further elucidated on the basis of embodiment examples shown schematically in the drawings, where
- Fig. 1: is a perspective view of a body-wearable medical device including a mounting base with an improved release mechanism;
- Fig. 2: is a perspective view of the medical device of fig. 1;
- Fig. 3: is an enlarged view of the release mechanism of the medical device; and
- Fig. 4: is a side view of the mounting base with a further embodiment of the release mechanism.

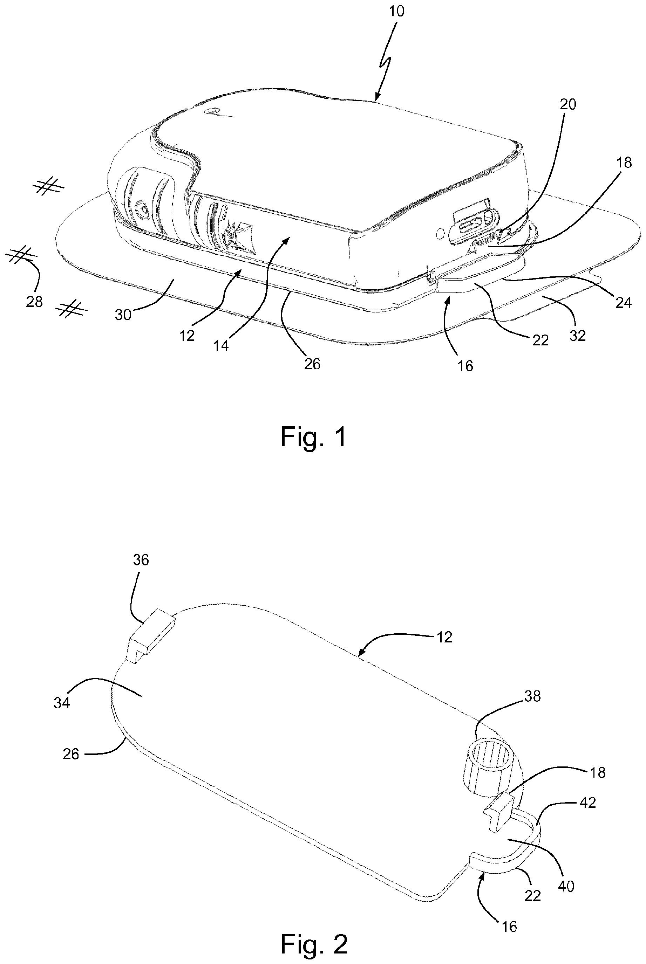

Referring to fig. 1, a medical device 10 comprises a mounting base or plate 12, a medical module 14 in the form of a body-worn patch pump for administration of insulin and a release mechanism formed as a snap connector 16 which is adapted to releasably attach the medical module 14 on the mounting plate 12 e.g. for first time assembly or removal in case of temporarily non-use or replacement. For this purpose, the snap connector 16 comprises a latch 18 that engages with a recess or notch 20 in a sidewall of the medical module 14 and a release tap or flap 22 that can be pushed down by the user or patient into a bottom free space or clearance 24 such that the flap 22 is allowed to move prior to a skin contact.

The mounting plate 12 has a bottom side 26 which is attachable at an infusion site to the skin 28 of the user via a self-adhesive pad 30 which is protected on its adhesive side by a peel-off foil 32.

As can be seen best in fig. 2, the mounting plate 12 has a top or upper side 34 which in use faces away from the skin 28 and supports the medical module 14. The latch 18 and a securing hook or bracket 36 are provided at opposite ends of the oblong mounting plate 12 as upstanding elements on the upper side 34. An upstanding hollow fitting or socket 38 provides skin access for a cannula assembly (not shown).

At the joining area of the latch 18, the flap 22 is moulded onto the edge of the mounting plate 12, such that it projects outwardly and is tilted upwards away from the skin 28, as will be explained in more detail below. Advantageously, the flap 22 has a flat base portion 40 and an upstanding circumferential rim 42 on the free outer edge of the base portion facing away from the skin 28.

The mounting plate 12 including its attached elements, 18,22,36,38 may be formed as an integral one-piece plastic part, e.g. by injection-moulding.

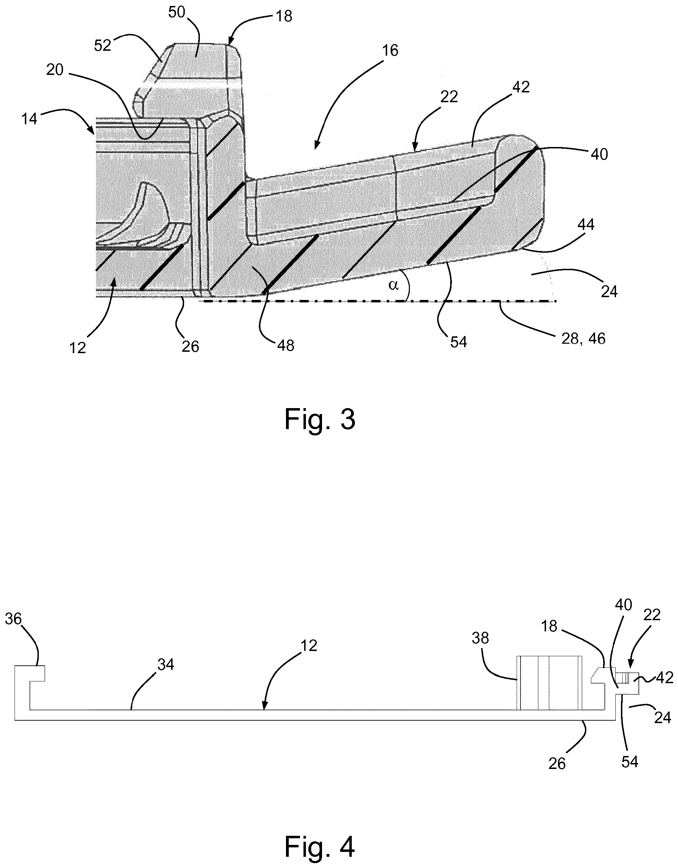

As further illustrated in fig. 3, the outer portion 44 of the release flap 22 maintains at its bottom side clearance 24, such that the release flap 22 is allowed to move under a force implied by finger pressure without interference, thus avoiding contact to the skin 28 or the self-adhesive pad 30 arranged thereupon. Then, the outer clearance distance to a plane 46 defined by the skin 28 (or, coinciding therewith, defined by the flat bottom side 26 of the mounting plate 12) should be about 5 mm, when viewed in direction perpendicular to the plane 46.

Fig. 3 further illustrates that the latch 18 has a hook portion 50 which is adapted to snap into the recess 20 of the medical module 14. The hook portion 50 is provided with an inlet chamfer 52 which guides the medical module 14 when introduced into the shown locking position. For this purpose, the inlet chamfer 52 is arranged at an acute angle with respect to the bottom side 26 of the mounting plate 12. For removal of the medical module 14, the latch 18 can be tilted outward (to the right in fig. 3) by actuation of the release flap 22 so as to disengage from the recess 20 in a release position.

In the embodiment of fig. 3, in order to allow a movement prior to an interfering skin contact, the distance of the connecting portion 48 of the flap 22 to the plane 46 should be smaller than the distance of the outer portion 44 to said plane 46. This can be realized arranging the flat bottom side 54 of the flap 22 under an acute angle α with respect to the skin 28 or plane 46. Preferably, said angle α is above 5°, e.g. around 10°.

In the embodiment of fig. 4, similar parts are indicated with similar reference signs as explained above. The main difference is in the arrangement of the release flap 22, which in this case on its bottom side runs at a distance parallel to the bottom side 26 of the mounting plate 12, thus maintain a free space with a constant clearance 24. In this configuration, the release flap 22 may be directly attached to the upstanding section of the latch 18.

## Claims

1. Skin-mountable medical device comprising:
a mounting plate (12) having a bottom side (26) attachable to the skin of a user preferably via an adhesive pad (30),
a medical module (14), in particular an analyte sensing device or a drug administration pump,
a snap connector (16) adapted to releasably attach the medical module (14) on the upper side (34) of the mounting plate (12) opposite to the bottom side (26), wherein the snap connector (16) has a latch (18) that is connected to the mounting plate (12) and engages with the medical module (14) in a locking position, wherein the snap connector (16) has a release flap (22) that can be actuated by the user to detach the medical module (14),
wherein the latch (18) can be tilted outward by actuation of the release flap (22) so as to disengage from the medical module (14) in a release position, and wherein the release flap (22) projects outwardly from the mounting plate (12),
**characterized in that** the release flap (22) has an outer portion (44) which at its bottom side (54) maintains a clearance (24) to avoid an interfering contact in use.

2. The medical device of claim 1, wherein the bottom side (54) of the mounting plate (12) lies in a plane (46), and the outer portion (44) of the release flap (22) is arranged at a clearance distance of at least 2 mm, preferably at least 5 mm above the plane (46).

3. The medical device of claim 1 or 2, wherein the release flap (22) has an inner portion (48) connected to the mounting plate (12), and the distance of the inner portion to a plane (46) which is defined by the bottom side (54) of the mounting plate (12) is smaller than the distance of the outer portion (44) to said plane (46).

4. The medical device according to any of claims 1 to 3, wherein the bottom sides (26,54) of the mounting plate (12) and of the release flap (22) each define a plane, and wherein said planes (46) include an acute angle (α).

5. The medical device of claim 4, wherein said acute angle (α) is at least 5°, preferably at least 10°.

6. The medical device of claim 1 or 2, wherein the bottom side (54) of the release flap (22) runs parallel to the bottom side (26) of the mounting plate (12).

7. The medical device according to any of claims 1 to 6, wherein the release flap (22) has an upstanding circumferential rim (42) which in use faces away from the skin.

8. The medical device according to any of claims 1 to 7, wherein the latch (18) has a hook portion (50) which is adapted to snap into a recess (20) of the medical module (14).

9. The medical device according to any of claims 1 to 8, wherein the snap connector (16) has an inlet chamfer (52) which is construed to guide the medical module (14) into a locking position, wherein the inlet chamfer (52) is arranged at an acute angle with respect to the bottom side (26) of the mounting plate (12) .

10. The medical device according to any of claims 1 to 8, further comprising a securing bracket (36) for attaching the medical module (14), wherein the securing bracket (36) and the snap connector (16) are arranged at opposite ends of the mounting plate (12).

11. The medical device according to any of claims 1 to 10, wherein the mounting plate (12) and the release flap (22) are formed as an integral, preferably injection-moulded plastic part.

12. The medical device according to any of claims 1 to 11, further comprising a fitting (38) arranged on the bottom plate to provide a skin access preferably for a cannula assembly of the medical module (14).

## Patentansprüche

1. Medizinische Vorrichtung, die auf der Haut anbringbar ist, umfassend:
eine Montageplatte (12) mit einer Unterseite (26), die vorzugsweise über ein Klebepad (30) an der Haut eines Benutzers befestigt werden kann,
ein medizinisches Modul (14), insbesondere ein Analyt-Messgerät oder eine Medikamentenverabreichungspumpe,
einen Schnappverbinder (16), der angepasst ist, um das medizinische Modul (14) auf der Oberseite (34) der Montageplatte (12) gegenüberliegend zu der Unterseite (26) lösbar zu befestigen,
wobei der Schnappverbinder (16) einen Riegel (18) aufweist, der mit der Montageplatte (12) verbunden ist und mit dem medizinischen Modul (14) in einer Verriegelungsposition in Eingriff steht,
wobei der Schnappverbinder (16) eine Entriegelungslasche (22) aufweist, die vom Benutzer betätigt werden kann, um das medizinische Modul (14) abzunehmen,
wobei der Riegel (18) durch Betätigung der Entriegelungslasche (22) nach außen geschwenkt werden kann, um sich in einer Freigabeposition von dem medizinischen Modul (14) zu lösen,
und wobei die Entriegelungslasche (22) von der Montageplatte (12) nach außen absteht,
**dadurch gekennzeichnet, dass** die Entriegelungslasche (22) einen äußeren Teil (44) besitzt, der an seiner Unterseite (54) einen Freiraum (24) aufrechterhält, um im Gebrauch einen Störkontakt zu vermeiden.

2. Medizinische Vorrichtung nach Anspruch 1, wobei die Unterseite (54) der Montageplatte (12) in einer Ebene (46) liegt, und der äußere Teil (44) der Entriegelungslasche (22) ist in einem Abstand von mindestens 2 mm, vorzugsweise mindestens 5 mm über der Ebene (46) angeordnet ist.

3. Medizinische Vorrichtung nach Anspruch 1 oder 2, wobei die Entriegelungslasche (22) einen mit der Montageplatte (12) verbundenen inneren Teil (48) aufweist, und der Abstand des inneren Teils zu einer durch die Unterseite (54) der Montageplatte (12) definierten Ebene (46) kleiner ist als der Abstand des äußeren Teils (44) zu dieser Ebene (46).

4. Medizinische Vorrichtung nach einem der Ansprüche 1 bis 3, wobei die Unterseiten (26, 54) der Montageplatte (12) und der Entriegelungslasche (22) jeweils eine Ebene definieren, und wobei die genannten Ebenen (46) einen spitzen Winkel (α) einschließen.

5. Medizinische Vorrichtung nach Anspruch 4, wobei der spitze Winkel (α) mindestens 5°, vorzugsweise mindestens 10° beträgt.

6. Medizinische Vorrichtung nach Anspruch 1 oder 2, wobei die Unterseite (54) der Entriegelungslasche (22) parallel zur Unterseite (26) der Montageplatte (12) verläuft.

7. Medizinische Vorrichtung nach einem der Ansprüche 1 bis 6, wobei die Entriegelungslasche (22) einen aufrechten Umfangsrand (42) aufweist, der im Gebrauch von der Haut wegweist.

8. Medizinische Vorrichtung nach einem der Ansprüche 1 bis 7, wobei der Riegel (18) einen Hakenabschnitt (50) aufweist, der dazu ausgebildet ist, in eine Aussparung (20) des medizinischen Moduls (14) einzurasten.

9. Medizinische Vorrichtung nach einem der Ansprüche 1 bis 8, wobei der Schnappverbinder (16) eine Einlaufschräge aufweist (52), die dazu ausgelegt ist, das medizinische Modul (14) in eine Verriegelungsposition zu bringen, wobei die Einlaufschräge (52) unter einem spitzen Winkel zur Unterseite (26) der Montageplatte (12) angeordnet ist.

10. Medizinische Vorrichtung nach einem der Ansprüche 1 bis 8, ferner umfassend einen Befestigungsbügel (36) zum Befestigen des medizinischen Moduls (14), wobei der Befestigungsbügel (36) und der Schnappverbinder (16) an gegenüberliegenden Enden der Montageplatte (12) angeordnet sind.

11. Medizinprodukt nach einem der Ansprüche 1 bis 10, wobei die Montageplatte (12) und die Entriegelungslasche (22) als einstückiges, vorzugsweise spritzgegossenes Kunststoffteil ausgebildet sind.

12. Medizinische Vorrichtung nach einem der Ansprüche 1 bis 11, ferner umfassend ein an der Bodenplatte angeordnetes Anschlussstück (38) zur Bereitstellung eines Hautzugangs vorzugsweise für einen Kanülenaufbau des medizinischen Moduls (14).

## Revendications

1. Dispositif médical pouvant être monté sur la peau comprenant :
une plaque de montage (12) dotée d'un côté inférieur (26) pouvant être fixé à la peau d'un utilisateur par l'intermédiaire d'un tampon adhésif (30),
un module médical (14), en particulier un dispositif de détection d'analyte ou une pompe d'administration de médicament,
un élément de liaison encliquetable (16) conçu pour fixer de manière amovible le module médical (14) sur le côté supérieur (34) de la plaque de montage (12) opposé au côté inférieur (26),
dans lequel l'élément de liaison encliquetable (16) est doté d'un verrou (18) qui est relié à la plaque de montage (12) et vient en prise avec le module médical (14) dans une position de verrouillage,
dans lequel l'élément de liaison encliquetable (16) est doté d'un volet de libération (22) qui peut être actionné par l'utilisateur pour détacher le module médical (14),
dans lequel le verrou (18) peut être incliné vers l'extérieur par l'actionnement du volet de libération (22) de manière à se dégager du module médical (14) dans une position de libération, et dans lequel le volet de libération (22) fait saillie vers l'extérieur à partir de la plaque de montage (12),
**caractérisé en ce que** le volet de libération (22) est doté d'une partie externe (44) qui, au niveau de son côté inférieur (54), maintient un dégagement (24) pour éviter un contact interférant lors de l'utilisation.

2. Dispositif médical selon la revendication 1, dans lequel le côté inférieur (54) de la plaque de montage (12) se situe dans un plan (46), et la partie externe (44) du volet de libération (22) est agencée à une distance de dégagement d'au moins 2 mm, de préférence d'au moins 5 mm au-dessus du plan (46).

3. Dispositif médical selon la revendication 1 ou 2, dans lequel le volet de libération (22) est doté d'une partie interne (48) reliée à la plaque de montage (12), et la distance de la partie interne à un plan (46) qui est défini par le côté inférieur (54) de la plaque de montage (12) est inférieure à la distance de la partie externe (44) audit plan (46).

4. Dispositif médical selon l'une quelconque des revendications 1 à 3, dans lequel les côtés inférieurs (26, 54) de la plaque de montage (12) et du volet de libération (22) définissent chacun un plan, et dans lequel lesdits plans (46) comportent un angle aigu (α).

5. Dispositif médical selon la revendication 4, dans lequel ledit angle aigu (α) est d'au moins 5°, de préférence d'au moins 10°.

6. Dispositif médical selon la revendication 1 ou 2, dans lequel le côté inférieur (54) du volet de libération (22) s'étend parallèlement au côté inférieur (26) de la plaque de montage (12).

7. Dispositif médical selon l'une quelconque des revendications 1 à 6, dans lequel le volet de libération (22) est doté d'un rebord circonférentiel vertical (42) qui est opposé à la peau lors de l'utilisation.

8. Dispositif médical selon l'une quelconque des revendications 1 à 7, dans lequel le verrou (18) est doté d'une partie de crochet (50) qui est conçue pour s'encliqueter dans un évidement (20) du module médical (14).

9. Dispositif médical selon l'une quelconque des revendications 1 à 8, dans lequel l'élément de liaison encliquetable (16) est doté d'un chanfrein d'entrée (52) qui est interprété pour guider le module médical (14) dans une position de verrouillage, dans lequel le chanfrein d'entrée (52) est agencé selon un angle aigu par rapport au côté inférieur (26) de la plaque de montage (12).

10. Dispositif médical selon l'une quelconque des revendications 1 à 8, comprenant en outre un support de fixation (36) permettant de fixer le module médical (14), dans lequel le support de fixation (36) et l'élément de liaison encliquetable (16) sont agencés à des extrémités opposées de la plaque de montage (12).

11. Dispositif médical selon l'une quelconque des revendications 1 à 10, dans lequel la plaque de montage (12) et le volet de libération (22) sont formés comme une pièce en plastique solidaire, de préférence moulée par injection.

12. Dispositif médical selon l'une quelconque des revendications 1 à 11, comprenant en outre un raccord (38) agencé sur la plaque inférieure pour fournir un accès cutané de préférence pour un ensemble canule du module médical (14).
